# EUROPEAN PATENT APPLICATION

(11) **EP 2 803 630 A1**
(43) Date of publication of application: **19.11.2014**
(21) Application number: 14001726.0
(22) Date of filing: 15.05.2014
(51) Int. Cl.: B67C 3/22, B08B 9/32, F16K 11/076

(54) **Manifold for conveying a sterilizing fluid inside empty articles**

(30) Priority: 15.05.2013 EP 13167932
(71) Applicant: Sidel S.p.A. Con Socio Unico, 43126 Parma (IT)
(72) Inventor: Vincenzo, Cero, 43126 Parma (IT)

(57) **Abstract**

There is disclosed a manifold (10) for conveying a sterilizing fluid inside a empty articles (2) movable about an axis (A), comprising: a stator (25), which defines at least one first duct (28) and a second duct (28) for the sterilizing fluid, each being connectable to a source of sterilizing agent through a first opening (27), and each comprising a second opening (24); a rotor (35), which defines one third duct (38a, 38b) and a fourth duct (38b, 38a) for sterilizing fluid; each third and fourth duct (38a, 38b) having a third opening (39) and a fourth opening (40); and a mask (45) interposed between first ducts (28) and third duct (38a, 38b) and configured to allow sterilizing agent to flow from first ducts (28) to fourth duct (38a, 38b); first duct and second duct (28) extend, at second opening (24), radially to said axis (A).

## Description

The present invention relates to a manifold for conveying a sterilizing fluid inside empty articles, in particular containers filled with a pourable food product.

Article-handling machines are known which comprise a plurality of units for carrying out respective operations on articles, in particular containers filled with a pourable food product.

In greater detail, the known article-handling machines substantially comprise:
- a sterilizing unit for sterilizing empty containers;
- a rinsing unit for rinsing containers;
- a filling unit for filling containers with a pourable food product;
- a capping unit for capping the containers; and
- a labelling unit for applying labels onto respective containers.

The sterilizing unit is adapted to inject a sterilizing agent, in particular a solution of peracetic acid, inside the empty containers and upstream of the rinsing unit.

In detail, the sterilizing unit comprises:
- one or more first wheel which rotates about an axis, and each comprises a plurality of jaws movable between an open position in which they receive/release respective containers and a closed position in which they convey the containers;
- one or more second wheel, known as distributors, which is coaxial with and arranged below the respective first wheel, and which comprises a plurality of nozzles arranged below respective jaws of the respective first wheel;
- a source of the sterilizing agent; and
- a manifold which is fluidly interposed between the source and the nozzles.

The first wheel is fed with containers to be sterilized at an inlet station, conveys the container along an arc-shaped path around the axis, and feeds an outlet station with the sterilized containers.

The containers are conveyed by the first wheel with respective mouths arranged downwardly and towards respective nozzles.

Due to the fact that the path extends for an arch which is less than 360 degrees, only a certain number of jaws convey respective containers.

The manifold is adapted to feed the sterilizing agent only to a certain number of nozzles, i.e. to those nozzles which are below to the containers conveyed by the first wheel.

On the other hand, the manifold is configured for preventing the sterilizing fluid from flowing from the source to the nozzles, which are arranged below the empty containers, in order to avoid any waste of sterilizing agent.

The manifold of the known sterilizing unit substantially comprises:
- a stator which is stationary relative to the axis and which defines an axial first duct in fluid connection with the source;
- a rotor, which rotates about the axis together with the second wheel and defines a plurality of second ducts in fluid connection with respective nozzles; and
- a stationary mask, which allows the fluid connection between the first duct and a given number of second ducts and prevents the fluid connection between the first duct and the remaining second ducts.

In detail, the mask is eccentric relative to the axis.

Due to the rotation of the rotor, only some second ducts - and therefore only some corresponding nozzles - are in fluidic connection with an outlet stretch of the mask. In particular, the mask puts in fluidic connection the source and only those nozzles which are arranged in the arch along which containers are conveyed by the first wheel

Furthermore, the known manifold comprises:
- a plurality of bearings, for example with ball bearings, which rotatably support the rotor on the stator; and
- a plurality of sealing elements, which protect the bearings from the sterilizing agent.

The Applicant has found that, when the sealing elements are worn out, the sterilizing agent contacts and, therefore, corrodes the bearings.

In order to prevent the resulting galling of the rotor and the stator, the manifold is generally replaced quite often, for example each three or four months depending on the throughput of the sterilizing unit.

A need is felt within the sector to avoid the above-described galling and to accordingly increase the life-time of the manifold.

Furthermore, a need is felt within the sector to reduce the weight and the cost of the manifold.

Finally, the mask is kept in position with the rotating part by an O-ring. Accordingly, when the mask is worn out, the sterilizing agent can flow also in the nozzles which are not arranged below the containers. This causes the waste of the sterilizing agent outside the arch of the path, along which the containers are conveyed.

A need is therefore felt within the industry to ensure that the wear of the rotor does not impair the sealing function of the mask.

It is an object of the present invention to provide a manifold for conveying a sterilizing fluid inside empty articles, which meets at least one of the above requirements.

The aforementioned object is achieved by the present invention as it relates to a manifold for conveying a sterilizing fluid inside empty articles, as claimed in claim 1.

One preferred embodiments is hereinafter disclosed for a better understanding of the present invention, by way of non-limitative example and with reference to the accompanying drawings, in which:
- Figure 1 is a perspective view of the manifold in accordance with the present invention;
- Figure 2 is an axial transversal section of the manifold of Figure 1;
- Figure 3 is a perspective view of the manifold of Figures 1 and 2, with parts removed for clarity;
- Figures 4 and 5 are sections taken along lines IV-IV and V-V of Figure 2;
- Figure 6 shows a frontal view, with some sectioned components, of a sterilizing unit, in which the manifold of Figures 1 to 5 is incorporated; and
- Figure 7 is a top view of the sterilizing unit of Figure 6.

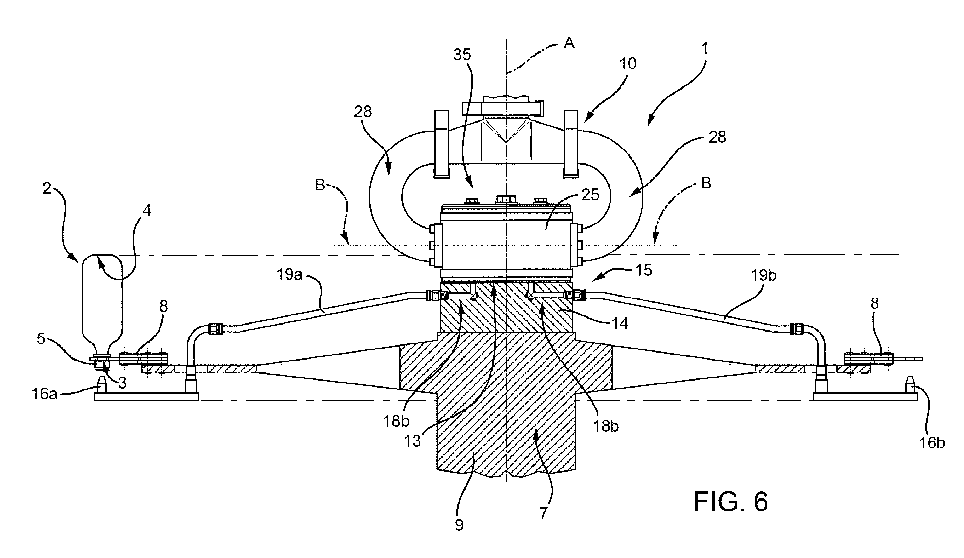

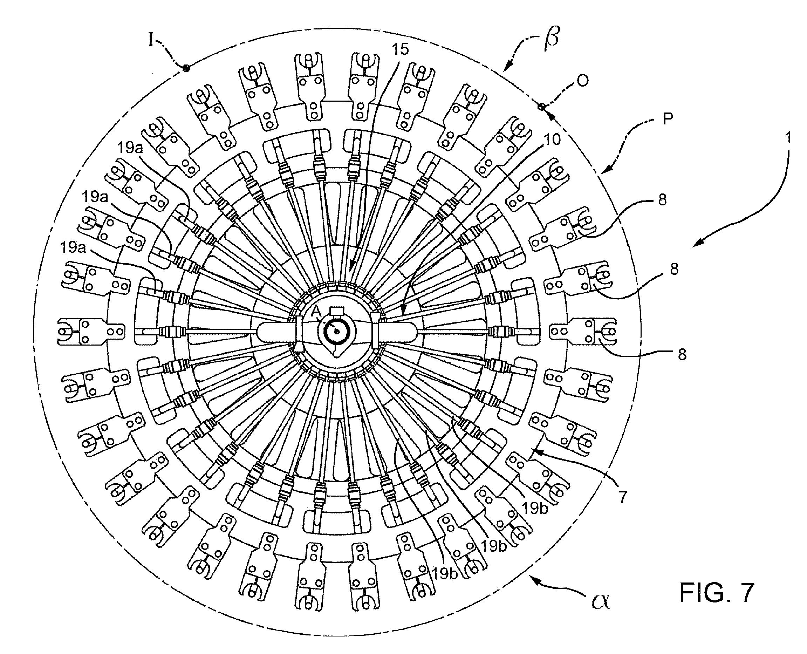

With reference to Figures 6 and 7, numeral 1 indicates a sterilizing unit for sterilizing empty containers 2 filled with a pourable food product.

Non-limitative examples of the pourable food product are fruit juices, tea, beer or energy drinks.

Unit 1 is adapted to inject a sterilizing agent, peracetic agent in the embodiment shown, inside empty containers 2 (shown only in Figure 6), before the latter are filled the food product.

Unit 1 is incorporated in an article-handling machine which may comprise one or more of the following:
- a rinsing unit (not-shown) for removing the sterilizing agent from empty containers 2 before the filling thereof and arranged downstream of unit 1;
- a capping unit (not-shown) for applying a plurality of caps onto respective filled containers 2; and
- a labelling unit (not-shown) for applying a plurality of labels onto respective empty or filled containers 2.

In the embodiment shown in Figure 6, each container 2 substantially comprises:
- a mouth 3 adapted to allow the filling/pouring of the same container 2;
- a bottom wall 4 opposite to mouth 3; and
- a neck 5 arranged close to mouth 3.

Containers 2 may be made of glass or plastic.

Unit 1 extends about an axis A, vertical in the embodiment shown, and substantially comprises:
- a tank filled with the sterilizing agent;
- a wheel 7 which rotates about axis A, and which comprises a plurality of pairs of jaws 8 for receiving, conveying and releasing respective containers 2;
- a distributor wheel 15 which rotates about axis A together with wheel 7, and comprises a plurality of peripheral nozzles 16a, 16b arranged in axial correspondence with and below respective jaws 8; and
- a manifold 10 which selectively creates a fluidic connection between the tank and nozzles 16a and prevents the fluidic connection between the tank and second nozzles 16b.

In detail, wheel 7 comprises:
- a hub 9; and
- a plurality of angularly equi-spaced arms, which radially protrude from hub 9 and to which the pairs of jaws 8 are hinged to respective spokes of wheel 7 about respective vertical axes parallel to axis A.

In detail, wheel 7 receives containers 2 at an inlet station I, releases containers 2 at an outlet station O, and conveys containers 2 along an arch-shaped path P (Figure 7).

Path P has centre on axis A and extends from station I and station O.

Path P extends about axis A for an arch α which is less than 360 degrees.

Wheel 7 also moves along an arch β, which extends from station O to station I.

The sum of arch α and arch β is 360 degrees.

Each container 2, when travelling along path P and arch α, is arranged with respective mouth 3, which is arranged above and faces respective nozzle 16a and which is arranged below respective wall 4.

Each pair of jaws 8 is movable between an open configuration in which they grip neck 5 of respective container 2 and a closed configuration in which they are detached from neck 5 of respective container 2.

In particular, jaws 8 are in the open configuration when they receive respective containers 2 at station I and when they release respective containers 2 at station O. Jaws 8 are in the closed configuration when they convey containers 2 along path P.

Jaws 8 travelling along path P grip respective containers 2 whereas jaws 8 travelling from station O from station I do not grip any container 2.

Distributor wheel 15 comprises, in turn,
- a hub 14, which is axially interposed between manifold 10 and hub 9;
- a plurality of ducts 18a, 18b, which are defined by hub 14 and selectively connectable with the tank by means of manifold 10; and
- a plurality of conduits 19a, 19b which fluidly connect respective ducts 18a, 18b with respective nozzles 16a and nozzles 16b and, are arranged outside hub 14.

Hub 14 is bounded, on the side of manifold 10, by an annular surface 13 from which an axial connecting element 12 protrudes along axis A.

Ducts 18a, 18b and conduits 19a, 19b are angularly equi-spaced relative to hub 14.

Conduits 19a, 19b protrude from hub 14 substantially along respective directions, which are transversal with respect to axis A.

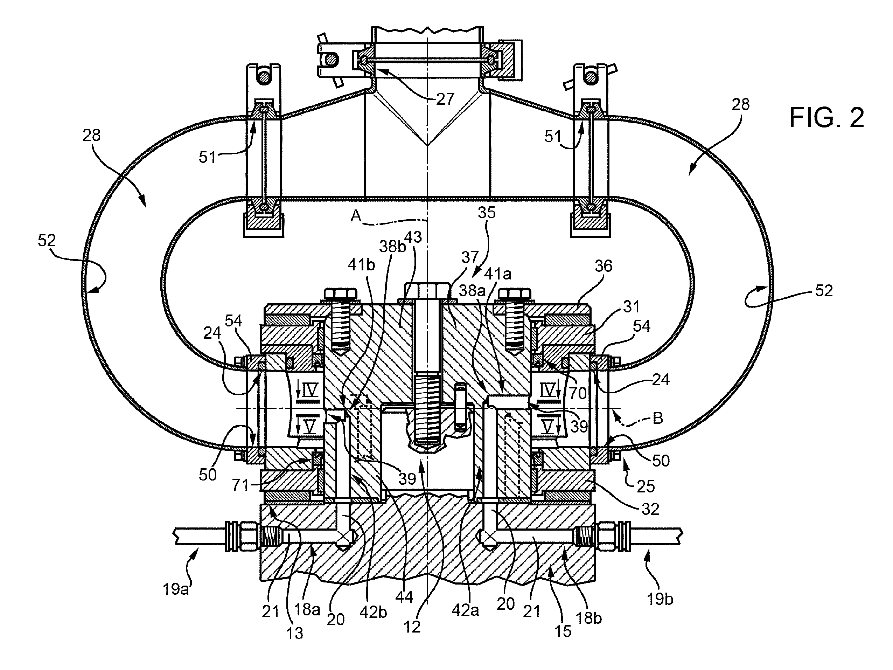

With reference to Figure 2, each duct 18a, 18b comprises an axial stretch 20 and a radial stretch 21, proceeding from manifold 10 towards conduits 19a, 19b.

Manifold 10 connects the tank with only nozzles 16a that are moving along arch α of path P and that, therefore, are arranged below respective containers 2 (Figure 6).

Furthermore, manifold 10 prevents nozzles 16b-those which are travelling outside path P and from station O to station I along arch β - from being fluidly connected with the tank.

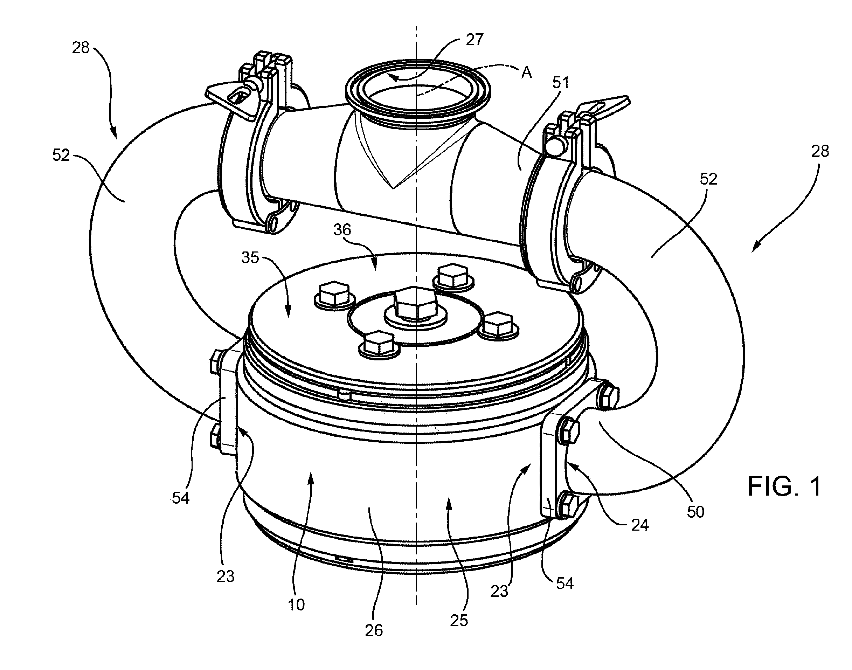

With reference to Figures 1 to 5, manifold 10 comprises:
- a stator 25 which is stationary relative to axis A;
- a rotor 35 which rotates about axis A integrally with wheels 7, 15.

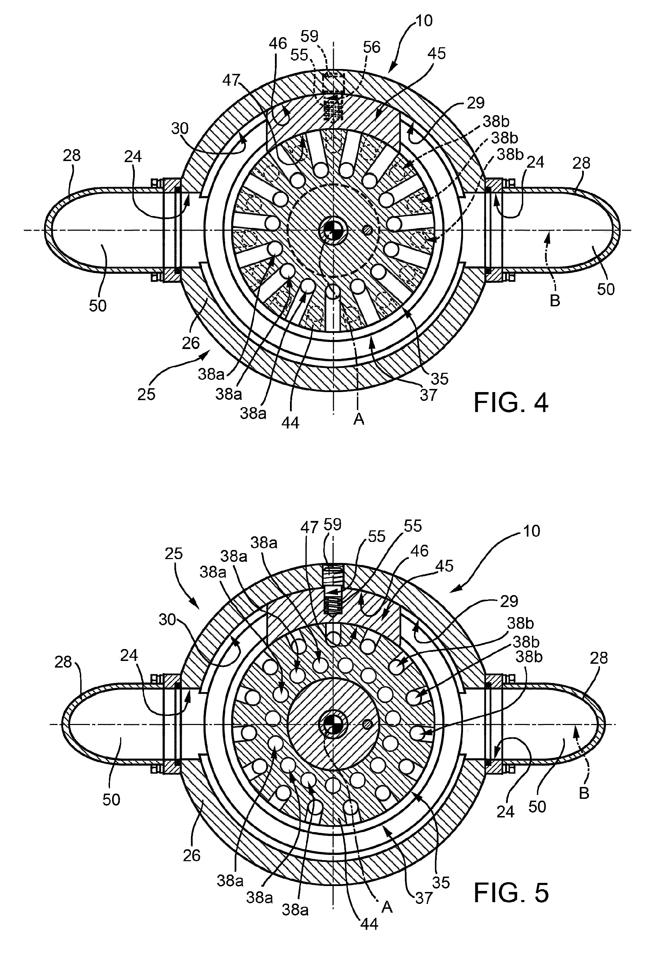

In detail, stator 25 comprises, in turn,:
- a tubular body 26 which houses rotor 35 and defines an annular groove 29 (Figures 4 and 5);
- a pair of annular flanges 31, 32 fixed respectively to a top and bottom surface of body 26 (figure 2);
- an inlet opening 27, which is arranged outside body 26 and extends about axis A and is fluidly connected with the tank; and
- a pair of ducts 28 which extends outside body 26, on opposite lateral sides of axis A, from opening 27 to

respective outlet openings 24.

Rotor 35 comprises, in turn,:
- a top flange 36 shaped as a circular crown having centre on axis A; and
- a body 37 which defines a plurality of ducts 38a, 38b connected, each, to a respective nozzle 16a, 16b.

Body 37 is fixed to connecting element 12 of distributor wheel 15, so that rotor 35 and wheel 15 rotate integrally to one another along axis A.

Body 37 comprises, in turn,:
- a half 43 to which flange 36 is fixed and axially spaced from flange 31; and
- a half 44 which defines ducts 38a, 38b.

Half 44 is hollow and houses connecting element 12.

Each duct 38a, 38b comprises an opening 39 which is open inside groove 29 and an opening 40, opposite to opening 39, and which is in correspondence of a relative duct 18a, 18b.

Ducts 38a, 38b are angularly equi-spaced about axis A.

Still more precisely, each duct 38a, 38b comprises, proceeding from groove 29 towards respective ducts 18a, 18b (Figure 4 and 5):
- a respective radial stretch 41a, 41b which defines relative opening 39; and
- a respective axial stretch 42a, 42b which defines respective opening 40.

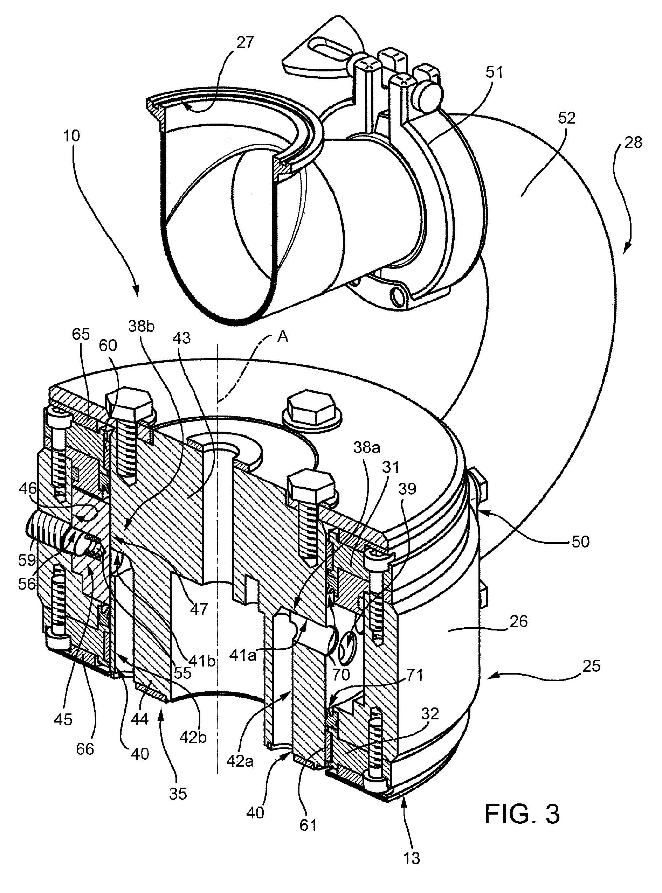

Furthermore, stretches 42b of ducts 38b are radially external relative to stretches 42a of ducts 38a (Figure 3).

Stretches 41b of ducts 38b are at a lower height than stretches 41a of ducts 38a.

It is important to stress that each duct 38a, 38b is associated to a respective either nozzle 16a or 16b, but each duct 38a is not perforce associated to a respective nozzles 16a and each duct 38b is not perforce associated to a respective nozzles 16b.

Manifold 10 also comprises a mask 45 which is stationary about axis A and is housed inside groove 29.

Mask 45 extends inside groove 29 for an arch, which is substantially equal to arch β of path P.

Mask 45 substantially comprises (Figure 4):
- a radially outer surface 46 which is pressed against a portion of a radially inner wall 30 of groove 29;
- a radially inner surface 47 which obstruct ducts 38a, 38b associated to nozzles 16b, thus preventing the sterilizing agent from reaching nozzles 16b.

In this way, for each angular position of rotor 35 and distributor wheel 15 about axis A, the sterilizing agent is prevented from flowing inside a group of ducts 38a, 38b and from reaching relative nozzle 16b, which move upstream of station O and downstream of station I along arch β.

Advantageously, ducts 28 have respective portions 50 which define respective openings 24 and extend radially to axis A.

In detail, openings 24 extend about respective axes B which are radial and, therefore, orthogonal to axis A.

Furthermore, openings 24 open inside groove 29.

As a result of the conformation of openings 24, the sterilizing agent enters groove 29 with a main component parallel to axes B and, therefore, radial to axis A.

Still more precisely, ducts 28 at first diverge from each other and then converge towards each other symmetrically to axis A, proceeding from body 26 towards openings 24.

Each duct 28 also comprises, proceeding from opening 27 towards relative opening 24,:
- a radial portion 51;
- a curved portion 52;
- radial portion 50 which defines relative opening 24, enters a portion 23 of body 26 and is fitted to a relative flange 54 of body 26.

Manifold 10 also comprises a pair of annular guide elements 60, 61 which guide rotor 35 in its rotation about axis A relative to stator 25 and centre rotor 35 on stator 25 (Figure 3).

In detail, guide element 60 is radially interposed between flange 31 of stator 25 and half 43 of rotor 35. Furthermore, guide element 61 is radially interposed between flange 32 of stator 25 and half 44 of rotor 35.

Manifold 10 also comprises a pair of support elements 65, 66 for supporting the axial load exerted by sterilizing agent flowing on manifold 10 (Figure 3).

In detail, support element 65 is annular and axially interposed between flange 31 of stator 25 and flange 36 of rotor 35.

Support element 66 is annular and is axially interposed between flange 32 of stator and surface 13 of distributor wheel 15.

Preferably, guide elements 60, 61 and/or support elements 65, 66 are made in plastic material, in particular a plastic material with low sliding friction coefficient, high wear resistance, high chemical corrosion resistance and very low thermal expansion coefficient.

Preferably, support elements 60, 61 are made in Peek.

Finally, manifold 10 comprises a pair of annular sealing elements 70, 71 which prevent any potential leakage of the sterilizing agent and thereby maintain a constant pressure in nozzles 16a.

In detail, sealing element 70 is radially interposed between a radial outer surface of half 43 of rotor 35 and portion 23 of body 26 receiving ducts 28, and is axially interposed between ducts 28 and guide element 60.

Sealing element 71 is radially interposed between a radial outer surface of half 44 of rotor 35 and portion 23 of body 26 receiving ducts 28, and is axially interposed between ducts 28 and guide element 61.

Preferably, manifold 10 also comprises a spring 55 (Figure 3) housed inside a seat 56 of mask 45 and a screw 59 for connecting mask 45 and body 26 of stator 25.

Spring 55 is fitted to groove 29 and loads mask 45 against rotor 35.

In the embodiment shown, spring 55 is helical and extends radially to axis A.

In use, wheel 7 and distributor wheel 15 rotate integrally about axis A.

Wheel 7 is fed with containers 2 to be sterilized at station I, conveys containers 2 along arch α and outputs sterilized containers 2 at station O.

In particular, containers 2 are conveyed by wheel 7 along path P with respective mouths 3 arranged below respective bottom wall 4.

As distributor wheel 15 rotates, nozzles 16a are conveyed along arch α and arranged below mouths 3 of respective containers 2. Differently, nozzles 16b are travelling from station O to station I along arch β, and are arranged below respective pairs of jaws 8 which are not gripping any container 2 (Figure 7).

Manifold 10 feeds the sterilizing agent to nozzles 16a only and prevents the sterilizing agent from reaching nozzles 16b.

In greater detail, stator 25 remains stationary relative to axis A while rotor 35 is driven in rotation by distributor wheel 15 about axis A.

The sterilizing agent flows from the tank to opening 27 and then flows along ducts 28. Then, the sterilizing agent radially enters groove 29 of manifold 10, through openings 24.

It is important to stress that, due to the fact that openings 24 extend about axes B radially to axis A, the pressure of the sterilizing agent inside manifold 10 results in a substantial null radial action along axes B and in a very slight axial action along axis A.

For each angular position of rotor 35 and distributor wheel 15, mask 45 prevents the sterilizing agent from reaching those ducts 38a, 38b which are temporary in contact with surface 47 of mask 45. In this way, mask 45 prevents the sterilizing agent from reaching nozzles 16b, which are associated to ducts 38a, 38b temporary closed by mask 45.

On the other hand, for each angular position of rotor 35 and distributor wheel 15, mask 45 allows the sterilizing agent to reach those ducts 38a, 38b which are not covered by mask 45 and that are, therefore, temporary in free communication with groove 29. In this way, the sterilizing agent flow through ducts 38a, 38b associated to nozzles 16a up to reach the nozzles 16a themselves.

Nozzles 16a travelling along path α can thus inject the sterilizing agent inside respective containers 2 travelling along path P.

On the contrary, nozzles 16b travelling along path β are not fed the sterilizing agent.

Guide elements 60, 61 allow the rotation of rotor 35 relative to stator 25 and centre rotor 35 relative to stator 25.

Support elements 65, 66 support the slight axial loads exerted by rotor 35 on stator 25 and of stator 25 on distributor wheel 15 respectively.

Sealing element 70, 71 protect guide elements 60, 61 from any potential leakage of sterilizing agent.

From an analysis of the features of manifold 10 made according to the present invention, the advantages it allows to obtain are apparent.

In particular, openings 24 extend about axis B, which are radial to axis A.

In this way, the pressure of the sterilizing agent in groove 29 generates a substantially null radial action and a slight axial action.

The slight axial action is due to the load loss in ducts 28 between opening 27 and openings 24.

Accordingly, rotor 35 may be supported on stator 25, by using guide elements 60, 61 and support elements 65, 66, instead of rolling bearing as in the introductory part of the present description.

In other words, the radial conformation of openings 24 results in the replacement of rolling bearings with components subject to sliding friction.

Guide element 60, 61 and/or support element 65, 66 allow rotation of rotor 35 relative to stator 25, even when sealing elements 70, 71 are not completely effective in preventing the leakage of the sterilizing agent.

Accordingly, the risk of galling manifold 10 is lowered and the life-time of manifold 10 is highly increased when compared with the manifold described in the introductory part of the present description.

Furthermore, due to the very limited axial action due to the pressure of sterilizing agent, guide element 60, 61 and/or support element 65, 66 can be made in plastic material, especially Peek.

Furthermore, spring 55, together with the pressure of the sterilizing agent inside groove 29, keeps mask 45 in contact with rotor 35.

In this way, even when mask 45 is worn out, there is no substantial leakage of the sterilizing agent.

Finally, the guide element 60, 61 and/or support element 65, 66 are far lighter than rolling bearing, thus dramatically reducing the final weight and cost of manifold 10, when compared with those of the solution described in the introductory part of the present description.

Finally, it is apparent that modifications and variants not departing from the scope of protection of the claims may be made to manifold 10.

## Claims

1. A manifold (10) for conveying a sterilizing fluid inside a plurality of empty articles (2), which are movable about an axis (A) and arranged along an arch (α) having centre on said axis (A) and extending for less than 360 degrees about said axis (A), comprising:
- a stator (25), which is stationary relative to said axis (A) and defines at least one first duct (28) and a second duct (28) for said sterilizing fluid; each said first duct (28) and second duct (28) being fluidly connectable, through a first opening (27), to a source of said sterilizing agent, and comprising a relative second opening (24), opposite to said first opening (27);
- a rotor (35), which may rotate relative to said stator (25) about said axis (A) and defines at least one third duct (38a, 38b) and a fourth duct (38b, 38a) for said sterilizing fluid; each said third duct (38a, 38b) and fourth duct (38b, 38a) having a respective third opening (39) and a respective fourth opening (40), opposite to said third opening (39);
- a mask (45) fluidly interposed between said second opening (27) of at least one said first duct (28) and said third opening (39) of one of said third duct and said fourth duct (38a, 38b), on the basis of the angular position of said rotor (35) about said axis (A), so as to prevent said sterilizing agent from flowing inside said one of said third or fourth duct (38a, 38b); said mask (45) being configured to allow said sterilizing agent to flow from said second duct (28) to the other one of said third duct or fourth duct (38a, 38b), on the basis of the angular position of said rotor (35) about said axis (A);
**characterized in that** said first duct and second duct (28) extend, at least at said second openings (24), radially to said axis (A).

2. The manifold of claim 1, **characterized by** comprising at least one centering element (60, 61), which is radially interposed between said rotor (35) and stator (25), onto which said rotor (35) slidingly rotates with friction, and which is adapted to centre said rotor (35) on said stator (25) relative to said axis (A).

3. The manifold of claim 1 or 2, **characterized by** comprising at least one support element (65, 66), which is axially sandwiched between said rotor (35) and stator (25), onto which said rotor (35) slidingly rotates with friction, and which is adapted to axially support said rotor (35) onto said stator (25).

4. The manifold of claims 2 or 3, **characterized in that** said centering element (60, 61) and/or said support element (65, 66) is/are made of a plastic material.

5. The manifold of any one of the foregoing claims, **characterized by** comprising an annular groove (29) which is fluidly in connection, on one side thereof, with said first duct (28) and second duct (28) and, on the opposite side thereof, with said third duct and fourth duct (38a, 38b);
said mask (45) extending inside said groove (29), covering said one of said third and fourth duct (38a, 38b) and leaving free said other one of said third and fourth duct (38a, 38b).

6. The manifold of claim 5, **characterized in that** said groove (29) is defined by said stator (25).

7. The manifold of any one of the foregoing claims, **characterized by** comprising elastic means (55) for keeping in position said mask (45) against said rotor (35), and in that said mask (45) is connected to said stator (25).

8. The manifold of claim 7, **characterized in that** said elastic means (55) are fitted to said stator (25) and extends radially to said axis (A).

9. The manifold according to any one of the foregoing claims, **characterized in that** each said third and fourth duct (38a, 38b) comprises:
- a first stretch (41a, 41b), which extends radially to said axis (A) and defines said third opening (39); and
- a second stretch (42a, 42b), which extends axially and defines said fourth opening (40).

10. The manifold of any one of claims 5 to 9, **characterized in that** said third opening (39) is open inside said groove (29).

11. The manifold of claim 9 or 10, **characterized in that** said second stretch (42a) of said third duct (38a) is arranged radially internal with respect to the second stretch (42b) of said fourth duct (38b).

12. The manifold of any one of claims 8 to 11, **characterized in that** said first stretch (41a) of said third duct (38a) is arranged axially higher than said first stretch (41b) of said fourth duct (38b).

13. A unit (1) for sterilizing a plurality of empty articles (2) before the filling thereof and which may move about an axis (A), comprising:
- a conveying wheel (6) which comprises a plurality of conveying elements (8) for conveying said articles (2) about said axis (A) and along said arch (α) from an inlet station (I) to an outlet station (O);
- an injecting wheel (15) which comprises a plurality of nozzles (16a, 16b) associated to respective conveying elements (8) and adapted to inject said sterilizing agent inside said respective containers (2), when travelling along said arch (α); and
- a manifold (10) according to any one of the foregoing claims and which is angularly integral with said injecting wheel (15) relative to said axis (A);
each said first duct and second duct (38a, 38b) being in fluid connection with one respective said nozzle (16a, 16b).
